# EUROPEAN PATENT SPECIFICATION

(11) **EP 1 132 009 B1**
(45) Date of publication and mention of the grant of the patent: **18.10.2006**
(21) Application number: 01610015.8
(22) Date of filing: 27.02.2001
(51) Int. Cl.: A23K 1/175, A23K 1/16

(54) **Natural, intestinally active feed additive**
Natürlicher, intestinal aktiver Futterzusatz
Additif naturel et intestinalement actif pour nourriture animale

(30) Priority: 29.02.2000 DK 200000325
(43) Date of publication of application: 12.09.2001
(73) Proprietor: Nor-Natur ApS, 2650 Hvidovre (DK)
(72) Inventor: Elgaard, Troels, 2660 Brondby Strand (DK)
(74) Representative: Zeuthen-Aagaard, Henrik

(56) References cited:
- EP-B- 0 835 120
- US-A- 4 140 690
- US-A- 4 820 739
- US-A- 5 565 211
- US-A- 5 908 634
- BERENGUER, ANTONIO ALVAREZ: "Sepiolite in the field of animal nutrition" PROCEEDINGS OF THE 'INDUSTRIAL MINERALS' INTERNATIONAL CONGRESS 5TH, 1983, pages 37-45, XP001002480 Madrid, Spain

## Description

### Technical Field

The invention relates to an intestinally active feed additive with natural active substances or substances identical to natural substances, a method of preparing the feed additive, a feed mixture including the additive as well as a method of breeding farm animals.

### Background Art

It is well-known to use antibiotic or chemotherapeutic substances as so-called growth-promoters in connection with the breeding of farm animals. During recent years there has been a focus on the problems caused by the widespread use of antibiotics or chemotherapeutics as growth-promoters. More and more pathogenic micro-organisms can develop a resistance to the antibiotics in question which accordingly become less effective. A risk applies of antibiotic residues remaining in carcasses with the result that human beings also consume antibiotics, which in turn may have the result that micro-organisms infecting human beings with diseases can develop a resistance. In addition, a risk applies of a consuming of antibiotic-resistant micro-organisms together with the meat products.

A continued use of antibiotics as growth-promoters may increase the risk of an epidemic among animals and/or human beings which cannot be combatted by means of antibiotics. Furthermore, one of the hitherto used growth-promoters has turned out to be carcenogenic as well.

In view of these problems attempts have been made to develop alternative methods so as to ensure a disease-free breeding of farm animals without the use of antibiotics. Furthermore the use of the classic growth-promoters in the pig production has already ceased in some countries, such as in Denmark.

This cessation of the use of conventional growth-promoters in connection with the production of piglets in the weight ratio of 6 to 35 kg has turned out to cause problems in up to 80% of all stocks. Typical symptoms are an increased treatment for diarrhoea and that the piglets do not flourish, and an increased frequency of infections, especially caused by *E*. *coli* or *Lawsonia*, as well as dysentery and diseases similar to dysentery.

In order to economize on the breeding of farm animals a demand for alternative solutions exists accordingly, said alternative solutions being capable of preventing or reducing these infections and diseases in farm animals while not being encumbered with the risk of the classic growth-promoters of developing a resistance in the micro-organisms. Such solutions can advantageously be based on natural substances or substances identical to natural substances. These substances should also have the same or preferably an improved effect on the growth and the utilization of the animal feed of the animals compared to the classic growth-promoters in order to ensure the acceptance of the farmer and of the animal feed industry.

It is a well-known fact that many plants include various functional and/or antibacterial substances, such as flavonoids, hydroxycymenes and terpenes, and it is also a well-known fact that such active substances from plant materials are utilized in drugs.

Thus US-PS No. 5,939,050 (corresponding to WO 98/44 926) (Optiva corp.; Lokanathan et al.) describes an antibacterial composition comprising at least two components, which together show a synergistic effect with respect to a minimum inhibitory concentration. Examples of the two components are inter alia lemon oil and *Rosmarinus officinalis*-oil. The composition is preferably used for dental hygiene.

FR-PS No. 2 618 670 (Philip GML) relates to a composition against dental disorders.

The composition comprises six components, the interaction of which results in a synergistic effect. Among the six components the following are mentioned: essential oils from lemon, oregano and thyme. The composition has inter alia an anti-inflammatory and anti-viral effect.

It is also known to use various plant compositions in feed additives. Thus, CN 1 077 349 (Thieling City Chinese Herbal Medicine; Yan, B.) relates to an additive for animal feed which includes micronutrients and Chinese herbal medicine with zeolite as carrier.

US-PS No. 5,565,211 (Rossi) describes a composition for improving the digestibility of feed for ruminants. The composition comprises a phenol derivative, a flavouring mixture and a carrier which can include rosemary powder among other substances.

WO 97/01348 (Nitsas) discloses pharmaceutical compositions. As active material these compositions include essential plant oils from plants of the genus *Origanum*, especially *Origanum hyrtum* and *Origanum heracleoticum*. However, it is rather cost-intensive to obtain such essential plant oils, which per se renders said essential plant oils unsuited for daily prophylactic use. For veterinary use these compositions are available as powders composed of 94% of CaCO₃, 1 % of tannine and 5% of oregano oil. Such a composition ensures a fast release of the active ingredients, which is rather disadvantageous in case of a daily prophylactic use.

A. A. Berenguer and R. P. Castells: "Sepiolite in the field of animal nutrition" Proceeding of the 5th "Industrial Minerals" International Congress, 1983, pages 37 - 45, Madrid, Spain, discloses the use of sepiolite as a carrier for a supplement of micro-ingredients, such as vitamins, minerals" and antibiotics, to be incorporated in small doses in animal feed. The function of this carrier is to homogenise the supplement and to avoid segregation of the correct components. The great sorptive capacity of sepiolite is mentioned but use as a means for slow release of liquid or fluid active components continuously trthroughout the gastro-intestinal tract of the animals is not disclosed.

It is well-known to formulate drugs in form of enteral preparations which can pass unaffected through the stomach and not release the active drug until they reach the intestine. The preparation can for instance be provided with an enteral coating which can resist the acid environment of the stomach and thereby protect the inner core with the active drug of the preparation, and which is not dissolved and/or decomposed until it reaches the basic environment of the intestine. Another type of enteral preparations implies that the active drug is mixed into a matrix which is not decomposed until it reaches the intestine. Such enteral preparations include usually rather cost-intensive active drugs, and apart from the drug per se these preparations involve a relatively high production price. Accordingly, such preparations are too expensive to be used as feed additives.

Literature reveals a multitude of proposals for improving the health, the growth and the utilization of the animal feed in connection with breeding of farm animals, but none of these proposals has turned out to be so effective that they can replace the classic growth-promoters, such as tylosin phosphate and virginiamycin, without simultaneously increasing the costs of the animal feed industry or the farmer.

It turned out surprisingly that it is possible to produce an intestinally active feed additive by using a plant material including natural, active substances or corresponding substances identical to natural substances in a preparation where the active ingredients in a liquid preparation have been sorbed, viz. adsorbed on and/or absorbed in a porous, mineral carrier material of a particle size defined in greater detail, and where said intestinally active feed additive can match the classic growth-promoters with respect to cost-related efficiency.

### Brief Description of the Invention

The invention relates to a natural intestinally active feed additive including natural substances or substances identical to natural substances, and which in addition to possible conventional adjuvants or additives include
a) an active component comprising a plant material or obtainable from the plant material, said plant material originating from plants of the genus *Citrus,* or being a material including corresponding active substances identical to natural substances, which active component comprises a chemically modified active substance obtainable from a by-product from the production of citrus juice and/or 0.05 to 10 g of flavonoids per 1000 kg animal feed, and
b) a porous, mineral carrier material based on hydrated magnesium and/or aluminium silicates and being of such a nature that at least 55% by weight thereof present a particle size of between 50 µm and 700 µm,
where the active component (a) in liquid or fluid form is sorbed on and/or in the mineral carrier material (b).

The invention also relates to a feed additive as described above wherein the active component comprises 0.05 to 10 g of flavonoids per 1000 kg of animal feed, preferably 0.1 to 7 g of flavonoids per 1000 kg of animal feed.

The invention relates furthermore to a method of producing the feed additive, said method being characterised by adsorbing and/or absorbing by way of a sorption process the optionally diluted active component on/in the porous, mineral carrier material.

Finally, the invention relates to a feed mixture for farm animals and including the ingredients of the feed additive according to the invention together with a conventional animal feed, preferably in an amount of 0.5 to 12 kg, more preferred 3 to 7 kg, of a feed additive per 1000 kg of feed mixture, as well as a method of breeding farm animals where said animals are fed with such a feed mixture.

The feed additive according to the invention results in an improved growth and an improved utilization of the animal feed in connection with the breeding of healthy, but also less healthy farm animals, and an improved flourishing of less healthy farm animals.

The extent of applicability of the invention appears from the following detailed description. The detailed description and the specific examples are merely included to illustrate the preferred embodiments.

### Detailed Description of the Invention

The intestinally active feed additive according to the invention is based on functionally and/or antibacterially active natural and/or identical to natural substances which are sorbed, i.e. adsorbed on and/or absorbed in a particular, not too fine-grained, porous mineral carrier material, which ensures in a surprisingly simple manner that the release of the active substances is initiated in the stomach, and that the substances are released continuously through the following portions of the intestinal system in such a manner that the active substances are released both in the small intestine and in the large intestine.

The favourable release pattern of the active substances ensures flourishing and healthy farm animals in a more natural way than the one obtained by the classic growth-promoters. Thus it is possible to obtain the same and even a higher daily growth than the one obtained with the classic growth-promoters while the resistance problems associated with said antibiotic growth-promoters can simultaneously be avoided.

It is possible to extract various active ingredients from plant material of plants of the genus *Citrus* by way of a conventional isolation. In specific cases the active substances from *Citrus* plants can advantageously be converted into other active substances already present in plant materials by way of a simple chemical modification. Thus the citrus plant material is a highly suited source of hydroxycymenes with an antibacterial activity and which can be obtained by way of oxidation and aromatisation of cyclic terpenes. An example of hydroxycymene is 2-hydroxy-p-cymene (carvacrol).

An additional group of active compounds which can be extracted from *Citrus* plant materials are flavonoids. Flavonoids are a class of compounds which are derivatives of flavone (= 2-phenyl-1,4-benzopyrone). In these derivatives, one or more of the H-atoms in flavone are substituted with hydroxy groups or methoxy groups. Flavonoids exist in live tissue, where they are mainly bonded to sugar molecules.

Flavonoids are widely spread in plant materials. In particular citrus fruits, especially lemon and grapefruit, have turned out to be suitable flavonoid sources.

Flavonoid-containing plant materials from *Citrus* include also other active substances, which like the flavonoids disclose a moderate, but wide-spectrum, antibacterial effect.

The active component (a) is in the present description and the claims to be construed as a material including one or more active substances, and which is a material obtained either from one or more plants of the genus *Citrus* or from a corresponding synthetically produced material identical to a natural material. When the active component derives from plants, it can be obtained from the parts of a plant containing the active substances by way of isolation in a conventional physical and/or chemical way, such as by extraction by means of a suitable solvent, such as an alcohol, and a subsequent processing into a suitable liquid sorbable form. As mentioned, some of the active substances obtained from plant material can be chemically modified into other natural active substances.

As it is a question of natural materials, the content of the active substances may vary. Therefore the amounts used in the feed additive to obtain the desired effect must be related to the content of active substances in the used material. For this purpose the content in the material of hydroxycymenes and/or flavonoids can for practical reasons be used as a marker of the general content in the material of active substances.

Parts of plants from citrus fruits, such as lemon and grapefruit and related types of citrus, are particularly useful as starting material for the active component in the feed additive according to the invention, both because these fruits include cyclic terpenes convertible into hydroxycymenes, and because these fruits have a relatively high content of limonoids and flavonoids. These substances are functionally active and capable of binding volatile nitrogen compounds and sulphur compounds formed in connection with the digestion of the animal feed. In addition, the flavonoids have a wide-spectrum, antibacterial effect.

The active component in the feed additive according to the invention obtained from plant material originating from plants of the genus *Citrus* is typically used in an amount of 100 to 500, preferably 125 to 300 g per 1000 kg of animal feed, calculated on the basis of a material including 3 % by weight of flavonoids.

Juice is produced by way of pressing citrus fruits from which the coloured shell inter alia containing essential oils has been removed. After pressing a residue containing solid matter is obtained as a waste material. This waste material resulting from the juice production is suitable as starting material for the active component in the feed additive according to the invention. Thus the solid-matter-containing residue can be extracted by means of alcohol or water and be adjusted to a liquid preparation having approximately 50% by weight of dry matter, as such a compound would begin to gelate when the dry matter content exceeds approximately 60% by weight.

The active component or a portion thereof may advantageously be in form of a concentrated and possibly chemically modified extract of the above residue material from the juice production, and this extract is preferably used in an amount of 125 to 175 g/1000 kg of animal feed.

Some of the active substances obtainable from citrus fruits are fluids per se, and accordingly they need not be present in a dissolved form in order to be sorbable on the porous mineral carrier material. For instance carvacrol (2-hydroxy-p-cymene) is a fluid unlike the isomeric compound thymol (3-hydroxy-p-cymene) which has a melting point of 51.5°C. In order to ensure a uniform distribution of the sorbed active substances, the liquid active substances are usually mixed with a sweet oil (edible oil) followed by a sorbing of the mixture on the carrier material. When concentrated liquid active substance, such as carvacrol, is used, the feed additive contains preferably 10 to 100 g, particularly preferred 25 to 60 g of active substance per 1000 kg of animal feed.

An essential feature of the natural, intestinally active feed additive according to the invention is that the active component is sorbed on the porous, mineral carrier material (b), which is based on hydrated magnesium and/or aluminium silicates. It turned out that when this carrier material presents a suitable, not too small particle size, then some time will indeed pass before the active component sprayed thereon in form of a fluid compound becomes sufficiently adsorbed and absorbed on and in the porous carrier material. In return, the active substances are also released from the carrier material with a suitable delay in such a manner that the active substances pass the stomach of the animal and are only released here to a minor extent. A release exclusively in the stomach implies that the active substances are decomposed to a great extent by the gastric juice. This delay has the effect that a quantity of the active substances are not released until they reach the intestine of the animals, and as it appears from the feeding experiments carried out said active substances are capable of improving the health of the animals by virtue of their antibacterial and other functional effects, which ensures an improved utilization of the animal feed and an improved growth, i.e. a reduced number of stable days.

Examples of carrier materials (b) are sepiolite and other clay minerals of the sepiolite-palygorskite family, as well as zeolites, such as klinoptilolite, and bentonite. Such carrier materials can adsorb and absorb, viz. sorb, the active substances and release said substances at a suitable speed throughout the digestive system of the animals. The carrier material should be of such a particle size that the main portion, such as at least 55 % by weight, preferably at least 60% by weight, particularly preferred at least 80% by weight, presents a particle size of between 50 µm and 700 µm, preferably 100 µm and 650 µm, such as between 300 µm and 600 µm.

A suitable carrier material is Sepiolite 30/60 approved to be used in feed substances designated EEC-No. E 562, and of which 91 to 97% by weight have a particle size between 300 and 600 µm. Sepiolite 30/60 is obtained in Spain and is available from NOR-FEED ApS, Hvidovre, Denmark.

The feed additive according to the invention may according to a particular embodiment include one or more additional active components containing any active substance well-known per se and approved to be used in animal feed, including also antibacterial plant materials from plants not belonging to the genus *Citrus,* as well as corresponding antibacterial substances identical to natural substances.

A plant material suitably forming part of an embodiment according the invention as a supplementary active component is plant material from the labiatae family (Lamiaceae), such as for instance *Origanum vulgare* (oregano), *Thymus vulgaris* (thyme), *Rosmarinus officinalis* (rosemary), *Mentha piperita* (peppermint) as well as *Mentha arvensis* (corn mint). These known aromatic plants disclose a supplemental effect because obviously they stimulate the appetite of pigs, and they include also various active compounds, such as hydroxycymenes and terpenes providing them with the desired functional and wide-spectrum, antibacterial effect.

The collection of plant material from the labiatae family and the recovery or isolation of the active substances from said plants are relatively cost-intensive procedures, and accordingly they cannot be used in practice as the sole active component in a feed additive to be used daily. On the other hand, the flavouring properties of the aromatic plants have the effect that the additive is very attractive to the farm animals, and accordingly an increased feed consumption can be expected.

An example of the recovery of the supplemental active component from plant materials is as follows: the recovery from thyme can be carried out by pressing the leaves followed by an extraction by means of alcohol, whereafter the alcohol is evaporated to obtain a sprayable, liquid preparation with a suitable dry-matter content.

The supplementary active component may advantageously be in form of oregano oil or thyme oil, which is preferably used in an amount of 15 to 500, preferably 50 to 100 g per 1000 kg of animal feed.

The recovery of oregano oil or thyme oil is relatively expensive per se. A less expensive method of utilizing the advantages found in admixing plant material from a plant of the labiatae family as a supplemental active component is to use dried, ground leaves from the plant.

The active component or components forming part of the feed additive according to the invention may include materials with a content of various functionally and/or antibacterially active substances, which together with the remaining active substances in said materials ensure the effect of the active component in the feed additive. It is assumed that in particular three groups of compounds present in varying quantities in the plants in question contribute more or less to the functional and/or antibacterial effects.

One group of active compounds is the antibacterial hydroxycymenes, especially carvacrol and thymol.

Plant materials from plants of the genus *Citrus* may have a high content of cyclic terpenes, which are active compounds per se, but which can also be converted into hydroxycymenes in a relatively simple manner. . Other sources of hydroxycymenes are plants of the labiatae family, such as oregano, thyme and peppermint.

The second group of active compounds is flavonoids, which, as mentioned above, are widely spread in plant materials, and in particular in citrus fruits, especially in lemon and grapefruit.

The third group of active compounds are some functionally active terpenes. Examples of such functionally active terpenes are pinene, cymene, cineol, borneol and campher, which are present inter alia in plants of the labiatae family, such as in oregano, thyme and peppermint, as well as the limonene which is present in *Citrus.*

It should be underlined that the plant materials applicable as the active component or components may include various active substances both within and outside the above three groups of compounds, and that individually these active substances can be assumed to be important for the effect of the feed additive according to the invention.

In order to ensure that the necessary active substances are present in the feed additive according to the invention, it is at present preferred to use natural plant materials as source of the active component. Based on the present knowledge, the hydroxycymenes identical to natural hydroxycymenes are apparently just as efficient as the hydroxycymenes isolated directly from plant materials, i.e. without being subjected to a chemical modification.

Generally speaking, when it is desired to use a synthetically produced plant material identical to natural plant materials it is necessary to determine the extent in which other suitable quantities of other active substances should be present in the active component. It can for instance be mentioned that materials recovered from *Citrus* in addition to the flavonoids also include other active antibacterial substances, such as for instance phlorine, as well as limonoids binding ammonia.

Limonoid-containing materials are natural binders of volatile nitrogen-containing and sulphur-containing compounds, and they improve the utilization by the animals of the nitrogen and fatty content in the animal feed. Therefore the limonoides have also an environmentally improving effect because a reduced amount of nitrogen and sulphur compounds is thereby liberated together with the urine and the faeces.

The active component or components are preferably based on a plant material. The components are usually present in the feed additive in liquid form produced by way of extraction of the active substances from the plant material by means of a suitable solvent, usually an alcoholic solvent followed by a complete or partial evaporation of said solvent as well as, if necessary, a dilution by means of a sweet oil (edible oil) acceptable in the animal feed. The content of the active substances in the resulting liquid active component can vary, but the content of flavonoids is typically in the range of from 2 to 4% by weight, whereas the total content of hydroxycymenes and terpenes is typically in the range of 50 to 98% by weight.

A suitable active component can in a financially favourable manner be obtained from the fruit mass of citrus fruits, especially lemon or grapefruit, on the basis of the pulp-containing residue remaining as a by-product after the production of juice, the active substances being extracted and optionally modified as described above.

For the production of the feed additive according to the invention, the optionally diluted active component is adsorbed on and/or absorbed in the porous, mineral carrier material.

According to a preferred embodiment the following procedure is followed:
i) the active component is prepared as a liquid preparation,
ii) the liquid preparation is sprayed onto the porous mineral carrier material while being tumbled, and
iii) the tumbling is continued after the spraying has been terminated until the antibacterial component has been sorbed to a sufficient degree on and/or in the carrier material.

The tumbling in steps (ii) and (iii) can for instance be carried out in a horizontal mixer.

The liquid preparation may advantageously be produced by way of extraction of plant material containing the active component by means of an aqueous or an organic solvent, whereafter the used solvent or a portion thereof is evaporated as required and a diluent acceptable in animal feed, such as a sweet oil, preferably a vegetable oil, such as rape oil, or an alcohol, such as glycerol or propylene glycol can optionally be added.

### Example 1

For the production of 1 kg of feed additive the following materials are used:

| | |
|---|---|
| Carvacrol, min. 98%* | 12.5 g |
| Danish rape oil | 50.0 g |
| Sepiolite 30/60^{™}** | 937.5 g |

| | |
|---|---|
| *Carvacrol, min 98 % is an essential oil extracted from citrus fruit by oxidation and aromatisation of the limonene followed by concentration; available from Nor-Feed ApS, Hvidovre, Denmark. **Sepiolite 30/60^{™} is a sepiolite product, were at least 88% by weight thereof have the particle size 300 to 600 µm; available from Nor-Feed ApS, Hvidovre, Denmark. | |

The carrier, Sepiolite 30/60, is placed in a horizontal mixer. The mixture of rape oil and carvacrol is sprayed onto this carrier material while the carrier material is tumbled. After the spraying, the tumbling is continued for up to 20 minutes.

The feed additive can be used as animal feed to for instance pigs. Usually an amount of 2 to 6 kg per 1000 kg of animal feed is used.

### Example 2

For the production of 1 kg of feed additive the following materials are used:

| | |
|---|---|
| Nor-Spice® M Liquid* | 37.5 g |
| Sepiolite 30/60^{™} | 962.5 g |

| | |
|---|---|
| *Nor-Spice® M Liquid is a water-based citrus extract; available from Nor-Feed ApS, Hvidovre, Denmark. | |

The carrier, Sepiolite 30/60, is placed in a horizontal mixer. Nor-Spice® M Liquid is sprayed thereon while the carrier is tumbled. After the spraying, the tumbling is continued for up to 20 minutes.

The feed additive can be used as animal feed for for instance pigs. Usually an amount of 3 to 8 kg of additive is used per 1000 kg of animal feed.

### Example 3

For the production of 1 kg of feed additive the following materials are used:

| | |
|---|---|
| Nor-Spice® AB Liquid* | 50.0 g |
| Sepiolite 100^{™}** | 940.0 g |
| Orange oil | 5.0 g |
| Orange water phase | 5.0 g |

| | |
|---|---|
| *Nor-Spice® AB Liquid is a flavonoid-containing concentrated by-product from the production of lemon juice or grapefruit juice. **Sepiolite 100^{™} is a fine-grained sepiolite product, where at least 90% by weight thereof have a particle size smaller than 125 µm; available from Nor-Feed ApS, Hvidovre, Denmark. | |

The carrier, Sepiolite 100, is placed in a horizontal mixer. A mixture of Nor-Spice® AB Liquid and orange-water phase is sprayed thereon while the carrier is tumbled. After this spraying, orange oil is sprayed thereon. After the spraying, the tumbling is continued for up to 20 minutes.

The feed additive can be used as animal feed for for instance pigs. Usually an amount of 3 to 10 kg of additive is used per 1000 kg of animal feed.

### Example 4

For the production of 4 kg of feed additive the following materials are used:

| | |
|---|---|
| Carvacrol | 25.0 g |
| Nor-Spice® M Liquid | 100 g |
| Propylene glycol | 150 g |
| Sepiolite 30/60^{™} | 3,725 g |

The carrier, Sepiolite 30/60, is placed in a horizontal mixer. A mixture of carvacrol and Nor-Spice® M Liquid is sprayed thereon while the carrier is tumbled. Then propylene glycol is sprayed thereon. After the spraying, the tumbling is continued for up to 20 minutes.

The feed additive can be used as animal feed for for instance pigs. Usually an amount of 3 to 7 kg, especially 5 kg is used per 1000 kg of animal feed.

### Example 5

For the production of 4 kg of feed additive the following materials are used:

| | |
|---|---|
| Nor-Spice® M Liquid | 150 g |
| Dried, ground leaves of thyme | 200 g |
| Propylene glycol | 160 g |
| Sepiolite 100^{™} | 3,490 g |

The carrier, Sepiolite 100, is placed in a horizontal mixer. A mixture of Nor-Spice® M Liquid and propylene glycol is sprayed thereon while the carrier is tumbled. After the spraying, the tumbling is continued for up to 20 minutes. Finally, the dried, ground leaves of thyme is admixed.

The feed additive can be used as animal feed for for instance pigs. Usually an amount of 3 to 7 kg of additive is used per 1000 kg of animal feed.

### Example 6

For the production of 4 kg of feed additive the following materials are used:

| | |
|---|---|
| Carvacrol | 50 g |
| Rape oil | 200 g |
| Sepiolite 60/100^{™}* | 3,750 g |

| | |
|---|---|
| *Sepiolite 60/100^{™} is a sepiolite product, where at least 57% by weight have a particle size between 100 and 300 µm; available from Nor-Feed ApS, Hvidovre, Denmark. | |

The carrier, Sepiolite 60/100, is placed in a horizontal mixer. The carvacrol is mixed with the rape oil, and this mixture is sprayed onto the sepiolite while the carrier is tumbled. After the spraying, the tumbling is continued for up to 20 minutes.

The feed additive can be used as animal feed for for instance pigs. Usually an amount of 3 to 7 kg of additive is used per 1000 kg of animal feed.

### Example 7

For the production of 4 kg of feed additive the following materials are used:

| | |
|---|---|
| Nor-Spice® M Liquid | 200 g |
| Sepiolite 60/100^{™} | 3,800 g |

The carrier, Sepiolite 60/100^{™}, is placed in a horizontal mixer. Nor-Spice® M Liquid is sprayed onto the sepiolite while the carrier is tumbled. After the spraying, the tumbling is continued for up to 20 minutes.

The feed additive can be used as animal feed for for instance pigs. Usually an amount of 3 to 7 kg is used per 1000 kg of animal feed.

### Example 8

For the production of 1 kg of feed additive the following materials are used:

| | |
|---|---|
| Carvacrol | 10 g |
| Nor-Spice® AB Liquid | 50 g |
| Sepiolite 100^{™} | 940 g |

The carrier, Sepiolite 100^{™}, is placed in a horizontal mixer. Nor-Spice® AB Liquid and carvacrol is sprayed onto the sepiolite while the carrier is tumbled. After the spraying, the tumbling is continued for up to 20 minutes. **Feeding experiments**

In order to test the feed additive according to the invention, a number feeding experiments have been carried out. These feeding experiments were performed on healthy and sound pigs. Within the agriculture it is widely held that additives contributing to an increased growth of healthy animals also have an effect on less healthy or even sick animals.

### Feeding experiment 1

A feeding experiment has been carried out on pigs by means of the feed additive according to Example 6. The feeding experiment lasted for 21 days. As illustrated in the Table, this test period was divided into two periods, viz. a first period of 10 days and a second period of 11 days.

A control group of 23 pigs were fed with a mixture without a growth-promoter and included 37% of barley, 37% of wheat and 26% of supplementary feed of the brand "Soya Super" available from "Aarhusegnens Andel". The test group of 23 pigs were fed with this animal feed admixed the feed additive of Example 6 in an amount of 4 kg/1000 kg of animal feed. Both groups of pigs were healthy and sound at the beginning and the end of the experiment.

The results of the feeding experiments appear from Table 1 below.

**Table 1**

| | Control group First/Second period | Test group First/Second period | Difference First/Second period % | Difference The entire period % |
|---|---|---|---|---|
| Average weight at the beginning (kg) | 25.7/31.0 | 25.3/30.8 | -1.6/-0.6 | |
| Growth/day/pig (kg) | 0.53/0.591 | 0.55/0.627 | +3.8/+6.1 | +5.1 |
| Feed consumption/pig/day (FEs) | 0.99/1.09 | 1.00/1.11 | +1.0/+1.5 | +1.4 |
| Feed consumption/kg growth (FEs) | 1.86/1.85 | 1.83/1.76 | -1.6/-4.9 | -3.8 |

It appears from Table 1 that it is possible to obtain a total growth per pig which is 5.1% higher for pigs fed with a feed mixture including a feed additive according to the present invention compared to the control group not having received said feed additive. Furthermore it appears that this weight increase is obtained in a more profitable manner because the feed consumption per kg of growth, measured by numbers of feed units (FEs; = foderenheder (feed units) for svine), is 3.8% lower for pigs fed with a feed mixture including a feed additive according to the invention compared to pigs not having received this feed additive.

It should be noted that the positive result is obtained despite the fact that the pigs participating in the experiment, viz. both the control group and the test group, were completely healthy during the entire experiment and were kept in fine stables. It must be assumed that even better results can be achieved in connection with less healthy stocks.

### Feeding experiment 2

A feeding experiment has been carried out on pigs by means of the feed additive according to Example 7. The feeding experiment lasted for 9 days. A control group of 23 pigs were fed with a mixture of 37% of barley, 37% of wheat and 26% of supplementary feed of the brand "Soya Super"^{™} available from "Aarhusegnens Andel". The test group of 23 pigs were fed with this animal feed admixed the feed additive of Example 7 in an amount of 4 kg/1000 kg of animal feed. Both groups of pigs were healthy and sound at the beginning and the end of the experiment. The experiment was started the day the pigs were received in the store pig stable.

The results of the feeding experiments appear from Table 2 below.

**Table 2**

| | Control group | Test group | Difference % |
|---|---|---|---|
| Average weight at the beginning (kg) | 24.4 | 24.4 | 0 |
| Growth/day/pig (kg) | 0.489 | 0.522 | +6.8 |
| Feed/pig/day (FEs) | 0.96 | 0.99 | +3.0 |
| Feed/kg growth | 1.97 | 1.86 | -5.6 |

It appears from Table 2 that it is possible to obtain a total growth per pig which is 6.8% higher for pigs fed with a feed mixture including a feed additive according to the present invention compared to the control group not having received said feed additive. Furthermore it appears that this weight increase is obtained in a more profitable manner because the feed consumption per kg of growth, measured by numbers of feed units (FEs), is 5.6% lower for pigs fed with a feed mixture including a feed additive according to the invention compared to pigs not having received this feed additive.

### Feeding experiment 3

A feeding experiment was carried out for 21 days (29 November to 20 December 2000) by feeding pigs being subject to a weaning by means of a weaning mixture of wheat, barley, choco/cakemix, fishmeal, rape seeds and fat of 1.24 FEs per kg. The control group received this weaning mixture without additive whereas the test group received the same animal feed with 5 kg of feed additive according to Example 8 per ton of animal feed. The results appear from Table 3.

**Table 3**

| | Control group | Test group | Difference % |
|---|---|---|---|
| Number of pigs | 26 | 25 | |
| Average weight at the beginning (kg) | 7.5 | 8.16 | |
| Average weight at the end (kg) | 10.92 | 11.80 | |
| Growth per pig (kg) | 3.42 | 3.64 | |
| Growth per day per pig (g) | 167 | 173 | +3.6 |
| Feed consumption per pig per day FEs | 0.34 | 0.35 | +2.9 |
| Feed per kg growth FEs | 2.05 | 2.00 | -2.4 |

### Feeding experiment 4

A feeding experiment was carried out for 21 days (6 December to 27 December 2000) by feeding pigs being subject to a weaning by means of a weaning mixture of wheat, barley, choco/cakemix, fishmeal, rape seeds and fat containing 1.24 FEs per kg. The control group received the feed mixture alone, whereas the test group received the same animal feed with 4 kg of feed additive according to Example 8 per ton of animal feed. The results appear from Table 4.

**Table 4**

| | Control group | Test group | Difference % |
|---|---|---|---|
| Number of pigs | 25 | 25 | |
| Average weight at the beginning (kg) | 8.68 | 8.60 | |
| Average weight at the end (kg) | 12.88 | 13.68 | |
| Growth per pig (kg) | 4.20 | 5.08 | |
| Growth per day per pig (g) | 200 | 242 | +21.0 |

## Claims

1. Natural intestinally active feed additive including natural substances or substances identical to natural substances, **characterised by** including in addition to possible conventional adjuvants or additives
a) an active component comprising a plant material or obtainable from the plant material, said plant material originating from plants of the genus *Citrus,* or being a material including corresponding active substances identical to natural substances, which active component comprises a chemically modified active substance obtainable from a by-product from the production of citrus juice and/or 0.05 to 10 g of flavonoids per 1000 kg of animal feed, and
b) a porous, mineral carrier material based on hydrated magnesium and/or aluminium silicates and being of such a nature that at least 55% by weight thereof present a particle size of between 50 µm and 700 µm,
where the active component (a) in liquid or fluid form is sorbed on and/or in the mineral carrier material (b).

2. Natural intestinally active feed additive according to claim 1 including natural substances or substances identical to natural substances, **characterised by** including in addition to possible conventional adjuvants or additives
a) an active component comprising a plant material or obtainable from the plant material, said plant material originating from plants of the genus *Citrus,* or being a material including corresponding active substances identical to natural substances, which active component comprises hydroxycymene and/or 0.05 to 10 g of flavonoids per 1000 kg of animal feed, and
b) a porous, mineral carrier material based on hydrated magnesium and/or aluminium silicates and being of such a nature that at least 55% by weight thereof present a particle size of between 50 µm and 700 µm,
where the active component (a) in liquid or fluid form is sorbed on and/or in the mineral carrier material (b).

3. Feed additive according to claim 1 or 2, **characterised by** further including a supplementary active component comprising a plant material or obtainable from the plant material, said plant material originating from plants of the family labiatae or being a material including corresponding substances identical to natural substances, where the supplemental active component is sorbed on and/or in the mineral carrier material.

4. Feed additive according to claim 1, 2 or 3, **characterised by** the porous mineral carrier material being of the type sepiolite, bentonite or zeolite, such as klinoptilolite.

5. Feed additive according to any one of the preceeding claims, **characterised by** the porous mineral carrier material being of such a nature that at least 60% by weight thereof have a particle size of between 100 µm and 650 µm.

6. Feed additive according to any preceding claim, **characterised by** the porous mineral carrier material being of such a nature that at least 60% by weight have a particle size of between 300 µm and 600 µm.

7. Feed additive according to any preceding claim, **characterised by** the active component being a plant material obtainable from the fruits lemon and/or grapefruit.

8. Feed additive according to claim 1 or 2, **characterised by** the active component comprising hydroxycymene obtainable by oxidation and aromatisation of limonene.

9. Feed additive according to claim 7 or 8, **characterised by** the active component comprising a combination of hydroxycymene and active substances from *Citrus,* which have not been chemically modified.

10. Feed additive according to claim 8 or 9, **characterised by** the active component comprising 20 to 100 g of hydroxycymene per 1000 kg of animal feed.

11. Feed additive according to claim 10, **characterised by** the active component comprising 35 to 60 g of hydroxycymene per 1000 kg of animal feed.

12. Feed additive according to claim 1, **characterised by** the active component comprising 0.05 to 10 g of flavonoids per 1000 kg of animal feed.

13. Feed additive according to claim 12, **characterised by** the active component comprising 0.1 to 7 g of flavonoids per 1000 kg of animal feed.

14. Feed mixture for farm animals comprising the ingredients of the feed additive according to any of the claims 1 to 13 together with a conventional animal feed.

15. Feed mixture according to claim 14 comprising 0.5 to 12 kg of a feed additive per 1000 kg of feed mixture.

16. Feed mixture according to claim 15 comprising 3 to 7 kg of a feed additive per 1000 kg of feed mixture.

17. A method of producing a natural, intestinally active feed additive according to any of the claims 1 to 13, **characterised by** the optionally diluted active component being adsorbed on and/or absorbed by way of a sorption process in the porous mineral carrier material.

18. Use of
a) an active component comprising a plant material or obtainable from the plant material, said plant material originating from plants of the genus *Citrus,* or being a material including corresponding active substances identical to natural substances, which active component comprises a chemically modified active substance obtainable from a by-product from the production of citrus juice and/or 0.05 to 10 g of flavonoids per 1000 kg of animal feed, and
b) a porous, mineral carrier material based on hydrated magnesium and/or aluminium silicates and being of such a nature that at least 55% by weight thereof present a particle size of between 50 µm and 700 µm,
where the active component (a) in liquid or fluid form is sorbed on and/or in the mineral carrier material (b) for the manufacture of a natural intestinally active feed additive for the breeding of farm animals.

## Patentansprüche

1. Natürlicher, intestinal aktiver Futterzusatz, der natürliche Substanzen oder zu natürlichen Substanzen identische Substanzen einschließt, **dadurch gekennzeichnet, daß** er zusätzlich zu möglichen herkömmlichen Adjuvantien oder Zusatzstoffen
a) eine aktive Komponente, die ein Pflanzenmaterial umfaßt oder aus dem Pflanzenmaterial erhältlich ist, wobei besagtes Pflanzenmaterial aus Pflanzen der Gattung *Citrus* stammt, oder ein Material ist, das entsprechende aktive Substanzen einschließt, die zu natürlichen Substanzen identisch sind, wobei diese aktive Komponente eine chemisch modifizierte aktive Substanz, die aus einem Nebenprodukt aus der Herstellung von Zitrussaft erhältlich ist, und/oder 0,05 bis 10 g Flavonoide pro 1.000 kg Tierfutter umfaßt, und
b) ein poröses, mineralisches Trägermaterial, das auf hydratisierten Magnesium- und/oder Aluminiumsilikaten beruht und von der Art ist, daß wenigstens 55 Gew.-% desselben eine Teilchengröße von zwischen 50 µm und 700 µm zeigen,
einschließt, wobei die aktive Komponente (a) in flüssiger oder fluider Form auf und/oder in dem mineralischen Trägermaterial (b) sorbiert ist.

2. Natürlicher, intestinal aktiver Futterzusatz nach Anspruch 1, der natürliche Substanzen und zu natürlichen Substanzen identische Substanzen einschließt, **dadurch gekennzeichnet, daß** er zusätzlich zu möglichen herkömmlichen Adjuvantien oder Zusatzstoffen
(a) eine aktive Komponente, die ein Pflanzenmaterial umfaßt oder aus dem Pflanzenmaterial erhältlich ist, wobei besagtes Pflanzenmaterial aus Pflanzen der Gattung *Citrus* stammt, oder ein Material ist, das entsprechende aktive Substanzen einschließt, die zu natürlichen Substanzen identisch sind, wobei besagte aktive Komponente Hydroxycymen und/oder 0,05 bis 10 g Flavonoide pro 1.000 kg Tierfutter umfaßt, und
(b) ein poröses, mineralisches Trägermaterial, das auf hydratisierten Magnesium- und/oder Aluminiumsilikaten beruht und von der Art ist, daß wenigstens 55 Gew.-% desselben eine Teilchengröße von zwischen 50 µm und 700 µm zeigen,
einschließt, wobei die aktive Komponente (a) in flüssiger oder fluider Form auf und/oder in dem mineralischen Trägermaterial (b) sorbiert ist.

3. Futterzusatz nach Anspruch 1 oder 2, **dadurch gekennzeichnet, daß** er weiter eine ergänzende aktive Komponente einschließt, die ein Pflanzenmaterial umfaßt oder aus dem Pflanzenmaterial erhältlich ist, wobei besagtes Pflanzenmaterial aus Pflanzen der Familie Labiatae stammt, oder ein Material ist, das entsprechende Substanzen einschließt, die zu natürlichen Substanzen identisch sind, wobei die ergänzende aktive Komponente auf und/oder in dem mineralischen Trägermaterial sorbiert ist.

4. Futterzusatz nach Anspruch 1, 2 oder 3, **dadurch gekennzeichnet, daß** das poröse mineralische Trägermaterial vom Typ Sepiolith, Bentonit oder Zeolith, wie etwa Klinoptilolith, ist.

5. Futterzusatz nach einem der vorangehenden Ansprüche, **dadurch gekennzeichnet, daß** das poröse mineralische Trägermaterial von der Art ist, daß wenigstens 60 Gew.-% desselben eine Teilchengröße von zwischen 100 µm und 650 µm zeigen.

6. Futterzusatz nach einem vorangehenden Anspruch, **dadurch gekennzeichnet, daß** das poröse mineralische Trägermaterial von der Art ist, daß wenigstens 60 Gew.-% eine Teilchengröße von zwischen 300 µm und 600 µm zeigen.

7. Futterzusatz nach einem vorangehenden Anspruch, **dadurch gekennzeichnet, daß** die aktive Komponente ein Pflanzenmaterial ist, das aus den Früchten Zitrone und/oder Grapefruit erhältlich ist.

8. Futterzusatz nach Anspruch 1 oder 2, **dadurch gekennzeichnet, daß** die aktive Komponente Hydroxycymen umfaßt, erhältlich durch Oxidation und Aromatisierung von Limonen.

9. Futterzusatz nach Anspruch 7 oder 8, **dadurch gekennzeichnet, daß** die aktive Komponente eine Kombination aus Hydroxycymen und aktiven Substanzen aus *Citrus* umfaßt, die chemisch nicht modifiziert worden sind.

10. Futterzusatz nach Anspruch 8 oder 9, **dadurch gekennzeichnet, daß** die aktive Komponente 20 bis 100 g Hydroxycymen pro 1.000 kg Tierfutter umfaßt.

11. Futterzusatz nach Anspruch 10, **dadurch gekennzeichnet, daß** die aktive Komponente 35 bis 60 g Hydroxycymen pro 1.000 kg Tierfutter umfaßt.

12. Futterzusatz nach Anspruch 1, **dadurch gekennzeichnet, daß** die aktive Komponente 0,05 bis 10 g Flavonoide pro 1.000 g Tierfutter umfaßt.

13. Futterzusatz nach Anspruch 12, **dadurch gekennzeichnet, daß** die aktive Komponente 0,1 bis 7 g Flavonoide pro 1.000 kg Tierfutter umfaßt.

14. Futtermischung für landwirtschaftliche Nutztiere, die die Inhaltsstoffe des Futterzusatzes nach einem der Ansprüche 1 bis 13 zusammen mit einem herkömmlichen Tierfutter umfaßt.

15. Futtermischung nach Anspruch 14, die 0,5 bis 12 kg eines Futterzusatzes pro 1.000 kg Futtermischung umfaßt.

16. Futtermischung nach Anspruch 15, die 3 bis 7 kg eines Futterzusatzes pro 1.000 kg Futtermischung umfaßt.

17. Verfahren zur Herstellung eines natürlichen, intestinal aktiven Futterzusatzes nach einem der Ansprüche 1 bis 13, **dadurch gekennzeichnet, daß** die fakultativ verdünnte aktive Komponente auf dem porösen mineralischen Trägermaterial adsorbiert und/oder mittels eines Sorptionsprozesses in diesem absorbiert wird.

18. Verwendung
a) einer aktiven Komponente, die ein Pflanzenmaterial umfaßt oder aus dem Pflanzenmaterial erhältlich ist, wobei besagtes Pflanzenmaterial aus Pflanzen der Gattung *Citrus* stammt, oder ein Material ist, das entsprechende aktive Substanzen einschließt, die zu natürlichen Substanzen identisch sind, wobei besagte aktive Komponente eine chemisch modifizierte aktive Substanz, die aus einem Nebenprodukt aus der Herstellung von Zitrussaft erhältlich ist, und/oder 0,05 bis 10 g Flavonoide pro 1.000 kg Tierfutter umfaßt, und
b) eines porösen mineralischen Trägermaterials, das auf hydratisierten Magnesium- und/oder Aluminiumsilikaten beruht und von der Art ist, daß wenigstens 55 Gew.-% desselben eine Teilchengröße von zwischen 50 µm und 700 µm zeigen,
wobei die aktive Komponente (a) in flüssiger oder fluider Form auf und/oder in dem mineralischen Trägermaterial (b) sorbiert ist, zur Herstellung eines natürlichen, intestinal aktiven Futterzusatzes für die Aufzucht von landwirtschaftlichen Nutztieren.

## Revendications

1. Additif alimentaire naturel à activité intestinale, contenant des substances naturelles ou des substances identiques à des substances naturelles, **caractérisé en ce qu'**il contient, en plus d'éventuels adjuvants ou additifs habituels,
a) un composant actif comprenant une matière végétale ou accessible à partir de cette matière végétale, laquelle matière végétale provient de plantes du genre *Citrus* ou est une matière contenant des substances actives correspondantes identiques à des substances naturelles, lequel composant actif comprend une substance active chimiquement modifiée, accessible à partir d'un sous-produit de la production de jus de citrus, et/ou de 0,05 à 10 g de flavonoïdes par tonne d'aliment pour animaux,
b) et un matériau minéral poreux servant de support, à base de silicates hydratés de magnésium et/ou d'aluminium, et d'une nature telle que, pour au moins 55 % de son poids, il se présente en particules de 50 à 700 µm de taille,
le composant actif (a) étant, sous forme liquide ou fluide, adsorbé sur et/ou absorbé dans le matériau support minéral.

2. Additif alimentaire naturel à activité intestinale, conforme à la revendication 1, contenant des substances naturelles ou des substances identiques à des substances naturelles, **caractérisé en ce qu'**il contient, en plus d'éventuels adjuvants ou additifs habituels,
a) un composant actif comprenant une matière végétale ou accessible à partir de cette matière végétale, laquelle matière végétale provient de plantes du genre *Citrus* ou est une matière contenant des substances actives correspondantes identiques à des substances naturelles, lequel composant actif comprend un hydroxycymène et/ou de 0,05 à 10 g de flavonoïdes par tonne d'aliment pour animaux,
b) et un matériau minéral poreux servant de support, à base de silicates hydratés de magnésium et/ou d'aluminium, et d'une nature telle que, pour au moins 55 % de son poids, il se présente en particules de 50 à 700 µm de taille,
le composant actif (a) étant, sous forme liquide ou fluide, adsorbé sur et/ou absorbé dans le matériau support minéral.

3. Additif alimentaire conforme à la revendication 1 ou 2, **caractérisé en ce qu'**il contient en outre un composant actif supplémentaire comprenant une matière végétale ou accessible à partir de cette matière végétale, laquelle matière végétale provient de plantes de la famille des labiées ou est une matière contenant des substances actives correspondantes identiques à des substances naturelles, lequel composant actif supplémentaire est adsorbé sur et/ou absorbé dans le matériau support minéral.

4. Additif alimentaire conforme à la revendication 1, 2 ou 3, **caractérisé en ce que** le matériau support minéral poreux est de type sépiolite, bentonite ou zéolite, comme de la klinoptilolite.

5. Additif alimentaire conforme à l'une des revendications précédentes, **caractérisé en ce que** le matériau support minéral poreux est d'une nature telle que, pour au moins 60 % de son poids, il se présente en particules de 100 à 650 µm de taille.

6. Additif alimentaire conforme à l'une des revendications précédentes, **caractérisé en ce que** le matériau support minéral poreux est d'une nature telle que, pour au moins 60 % de son poids, il se présente en particules de 300 à 600 µm de taille.

7. Additif alimentaire conforme à l'une des revendications précédentes, **caractérisé en ce que** le composant actif est une matière végétale accessible à partir de fruits, citrons et/ou pamplemousses.

8. Additif alimentaire conforme à la revendication 1 ou 2, **caractérisé en ce que** le composant actif comprend un hydroxycymène accessible par oxydation et aromatisation de limonène.

9. Additif alimentaire conforme à la revendication 7 ou 8, **caractérisé en ce que** le composant actif comprend une combinaison d'hydroxycymène et de substances actives de *Citrus* qui n'ont subi aucune modification chimique.

10. Additif alimentaire conforme à la revendication 8 ou 9, **caractérisé en ce que** le composant actif comprend 20 à 100 g d'hydroxycymène par tonne d'aliment pour animaux.

11. Additif alimentaire conforme à la revendication 10, **caractérisé en ce que** le composant actif comprend 35 à 60 g d'hydroxycymène par tonne d'aliment pour animaux.

12. Additif alimentaire conforme à la revendication 1, **caractérisé en ce que** le composant actif comprend 0,05 à 10 g de flavonoïdes par tonne d'aliment pour animaux.

13. Additif alimentaire conforme à la revendication 12, **caractérisé en ce que** le composant actif comprend 0,1 à 7 g de flavonoïdes par tonne d'aliment pour animaux.

14. Mélange alimentaire pour animaux de ferme, comprenant les ingrédients d'un additif alimentaire conforme à l'une des revendications 1 à 13, joints à un aliment habituel pour animaux.

15. Mélange alimentaire conforme à la revendication 14, comprenant 0,5 à 12 kg d'additif alimentaire par tonne de mélange alimentaire.

16. Mélange alimentaire conforme à la revendication 15, comprenant 3 à 7 kg d'additif alimentaire par tonne de mélange alimentaire.

17. Procédé de production d'un additif alimentaire naturel à activité intestinale, conforme à l'une des revendications 1 à 13, **caractérisé en ce que** l'on fait en sorte que le composant actif, éventuellement dilué, s'adsorbe sur et/ou soit absorbé dans le matériau support minéral poreux, par un processus de sorption.

18. Emploi, en vue de la fabrication d'un additif alimentaire natu-rel à activité intestinale, destiné à l'élevage d'animaux de ferme,
a) d'un composant actif comprenant une matière végétale ou accessible à partir de cette matière végétale, laquelle matière végétale provient de plantes du genre *Citrus* ou est une matière contenant des substances actives correspondantes, identiques à des substances naturelles, lequel composant actif comprend une substance active chimiquement modifiée, accessible à partir d'un sous-produit de la production de jus de citrus, et/ou de 0,05 à 10 g de flavonoïdes par tonne d'aliment pour animaux,
b) et d'un matériau minéral poreux servant de support, à base de silicates hydratés de magnésium et/ou d'aluminium, et d'une nature telle que, pour au moins 55 % de son poids, il se présente en particules de 50 à 700 µm de taille,
le composant actif (a) étant, sous forme liquide ou fluide, adsorbé sur et/ou absorbé dans le matériau support minéral (b).
